# EUROPEAN PATENT SPECIFICATION

(11) **EP 1 567 661 B1**
(45) Date of publication and mention of the grant of the patent: **22.09.2010**
(21) Application number: 03783727.5
(22) Date of filing: 21.11.2003
(51) Int. Cl.: C12Q 1/00, G01N 33/53, G01N 33/542, C12Q 1/04, C12Q 1/25, C12Q 1/34

(54) **METHODS, BIOSENSORS AND KITS FOR DETECTING AND IDENTIFYING FUNGI**
VERFAHREN, BIOSENSOREN UND KITS ZUM NACHWEIS UND ZUR IDENTIFIZIERUNG VON PILZEN
PROCEDES, BIOCAPTEURS ET NECESSAIRES DE DETECTION ET D'IDENTIFICATION DE CHAMPIGNONS

(30) Priority: 26.11.2002 US 429513 P
(43) Date of publication of application: 31.08.2005
(73) Proprietor: ECI Biotech Inc., Worcester, MA 01605 (US)
(72) Inventor: COLPAS, Gerard, J., Holden, MA 01520 (US); APPIAH, Barbara, A., Worcester, MA 01610 (US); SANDERS, Mitchell, C., West Boylston, MA 01583 (US)
(74) Representative: Kirkham, Nicholas Andrew
(86) International application number: PCT/US2003/037319
(87) International publication number: WO 2004/047614

(56) References cited:
- EP-A2- 0 286 434
- WO-A-02/10433
- WO-A-98/27223
- WO-A-98/42864
- WO-A-03/063693
- WO-A1-92/16648
- WO-A2-01/66790
- WO-A2-03/063693
- DE-A1- 19 617 338
- US-A- 4 693 972
- US-A- 5 098 830
- US-A- 5 449 612
- US-A- 5 512 429
- US-A- 5 516 638
- US-A- 5 695 928
- US-B2- 6 770 451
- POLACHECK I ET AL: "DISTRIBUTION OF AUTO LYSINS IN HYPHAE OF ASPERGILLUS-NIDULANS EVIDENCE FOR A LIPID MEDIATED ATTACHMENT TO HYPHAL WALLS" JOURNAL OF BACTERIOLOGY, vol. 135, no. 3, 1978, pages 741-747, XP008072318 ISSN: 0021-9193
- GIRAUD F ET AL: "Biodegradation Of Anthracene And Fluoranthene By Fungi Isolated From An Experimental Constructed Wetland For Wastewater Treatment" WATER RESEARCH, ELSEVIER, AMSTERDAM, NL, vol. 35, no. 17, December 2001 (2001-12), pages 4126-4136, XP004308089 ISSN: 0043-1354

### SEQUENCE LISTING

<110> Expressive Constructs, Inc.
   Colpas, Gerard J.
   Appiah, Barbara A.
   Sanders, Mitchell C.
<120> METHODS, BIOSENSORS, AND KITS FOR DETECTING AND IDENTIFYING MOLD AND FUNGI
<130> 3265.1007002
<150> 60/429,513
   <151> 2002-11-26
<160> 10
<170> FastSEQ for Windows Version 4.0
<210> 1
   <211> 12
   <212> PRT
   <213> Artificial Sequence
<220>
   <223> peptide sequence
<400> 1
   Lys Ala Ala His Lys Ser Ala Leu Lys Ser Ala Glu
   1 5 10
<210> 2
   <211> 14
   <212> PRT
   <213> Artificial Sequence
<220>
   <223> peptide sequence
<400> 2
   Lys His Leu Gly Gly Gly Ala Leu Gly Gly Gly Ala Lys Glu
   1 5 10
<210> 3
   <211> 14
   <212> PRT
   <213> Artificial Sequence
<220>
   <223> peptide sequence
<400> 3
   Lys His Leu Gly Gly Gly Ala Leu Gly Gly Gly Ala Lys Glu
   1 5 10
<210> 4
   <211> 11
   <212> PRT
   <213> Artificial Sequence
<220>
   <223> peptide sequence
<400> 4
   Ala Cys Cys Asp Glu Tyr Leu Gln Thr Lys Glu
   1 5 10
<210> 5
   <211> 11
   <212> PRT
   <213> Artificial Sequence
<220>
   <223> peptide sequence
<400> 5
   Ala Asp Thr Val Glu Pro Thr Gly Ala Lys Glu
   1 5 10
<210> 6
   <211> 13
   <212> PRT
   <213> Artificial Sequence
<220>
   <223> peptide sequence
<400> 6
   Lys Leu Pro His Lys Leu Ser Trp Ser Ala Asp Asn Pro
   1 5 10
<210> 7
   <211> 14
   <212> PRT
   <213> Artificial Sequence
<220>
   <223> peptide sequence
<400> 7
   Pro Val Pro Ser Thr Pro Pro Thr Pro Ser Pro Ser Thr Pro
   1 5 10
<210> 8
   <211> 14
   <212> PRT
   <213> Artificial Sequence
<220>
   <223> peptide sequence
<400> 8
   Lys Gln Asn Met Leu Ser Glu Val Glu Arg Ala Asp Thr Glu
   1 5 10
<210> 9
   <211> 11
   <212> PRT
   <213> Artificial Sequence
<220>
   <223> peptide sequence
<400> 9
   Lys His Leu Gly Gly Gly Gly Gly Ala Lys Glu
    1               5                   10
<210> 10
   <211> 12
   <212> PRT
   <213> Artificial Sequence
<220>
   <223> peptide sequence
<400> 10
   Asn Glu Ala Ile Gln Glu Asp Gln Val Gln Tyr Glu
    1               5                   10

## Description

### BACKGROUND OF THE INVENTION

Fungi are a kingdom of organisms that include mushrooms, mildews, molds, and yeasts. Fungi are very common and will grow almost anywhere sufficient moisture and food exist, usually causing the decomposition of whatever material they use for food. The materials most vulnerable to attack are water-damaged organic materials containing high amounts of cellulose and low amounts of nitrogen, such as paper and paper products, cardboard, ceiling tiles, wood and wood products, dust, paints, wallpaper, insulation materials, drywall, carpet, fabric, upholstery, hay, fiberboard, gypsum board, dust, lint, and jute. In addition to damaging the material on which they live, fungi can often be toxic to susceptible individuals, causing a wide range of diseases. Because of this, fungi cost the American economy billions of dollars every year in property damage and medical expenses.

Molds, such as *Stachybotrys chartarum* (also known as *Stachybotrys atra, Stachybotrys alternans*, or black mold), are of particular concern to building owners, the agricultural industry, the construction industry, and the health-care industry because they damage buildings, damage agricultural goods, and release materials that can be toxic to people that live and work wherever the molds are growing. To minimize this toxic threat, it is prudent practice to remove molds whenever they are detected.

It is believed that mold's toxicity is caused by their release of mycotoxins, including proteases which can cause severe reactions in those that are susceptible. Either breathing mold spores into the lungs or direct contact with the mold itself can cause an allergic reaction. Black mold has also been implicated in the deaths of small children in the United States.

Sensitization to molds is a known risk factor for life threatening exacerbations of asthma. The prevalence of asthma in children has increased ∼58%, along with a ∼78% increase in mortality rates, since about 1980. Molds of the genera *Alternaria* and *Cladosporium* are the most common molds to be found in the indoor environment and the most likely to be associated with asthma and allergies. Furthermore, developing an allergic reaction to one or both of these molds is considered the primary risk factor for life threatening asthma as an adult. (Zureik, M., et al., Sensitisation to Airborne Moulds and Severity of Asthma: Cross Sectional Study From European Community Respiratory Health Survey, BMJ 2002, 325,411)

Inhalation of mold spores has also been linked with diseases such as toxic pneumonitis, hypersensitivity pneumonitis, tremors, chronic fatigue syndrome, kidney failure, and cancer. It is believed that ∼9% of hospital-acquired (nosocomial) infections are caused by fungi. At least two mycotoxins, aflatoxins and ochratoxin A, have been classified by the National Toxicology Program as human carcinogens. One cause of Farmer Lung is thought to be *Thermoactinomycetes*, a fungi species found in moldy hay, straw, or grain dust, and has been extensively reported in many countries, including the United States. Additionally, mold spores are believed to be one of the leading causes of allergies and asthma in children. Currently, allergies are the 6th leading cause of chronic disease in the United States, costing the health care system about $18 billion annually.

Presently, the methods used to identify a species of mold include sampling, culturing, staining, and visual inspection, however, these methods are often uncertain or inaccurate. Furthermore, sampling and culturing are not reliable in determining an individual's health risk because an individual's susceptibility to mycotoxins can vary greatly.

Indentifying the *Stachybotrys* fungi is particularly problematic because a routine visual inspection is insufficient. The only reliable method of identifying *Stachybotrys* is to have a microscopic analysis performed by an accredited laboratory, which can take several days to complete.

WO98/2722 discloses that Cladosporium fungi can be detected in samples. But the document does not disclose peptides related to those mentioned in present independent claims 1, 7 or 13.

A system or biosensor that can detect the presence of fungi before it can cause significant property damage or adverse health effects would be advantageous.

### SUMMARY OF THE INVENTION.

In its broadest form the invention is defined in independent claims 1, 7 and 13:
A method for detecting the presence or absence of a *Cladosporium* fungus in a sample,
comprising the steps of:
   (a) contacting the sample with a detectably labelled peptide substrate consisting of an amino acid sequence selected from the group consisting of:
      KAAHKSALKSAE (SEQ ID NO: 1),
      ACCDEYLQTKE (SEQ ID NO: 4),
      KLPHKLSWSADNP (SEQ ID NO: 6), and
      KQNMLSEVERADTE (SEQ ID NO: 8);
         wherein said substrate is specific for at least one fungal protease produced by the fungus to be detected, and wherein the sample is contacted under conditions that result in modification of the substrate by the protease; and
   (b) detecting the modification or the absence of the modification of the substrate, wherein modification of the substrate indicates the presence of the fungus in the sample, and wherein the absence of modification of the substrate indicates the absence of the fungus in said sample.

Use of a biosensor for detecting the presence or absence of a *Cladosporium* fungus in a sample, said biosensor comprising a solid support and a detectably labelled peptide substrate consisting of an amino acid sequence selected from the group consisting of:
KAAHKSALKSAE (SEQ ID NO: 1),
ACCDEYLQTKE (SEQ ID NO: 4),
KLPHKLSWSADNP (SEQ ID NO: 6) and
KQNMLSEVERADTE (SEQ ID NO: 8);
   wherein said substrate is specific for a protease produced by the fungus to be detected, and wherein said detectably labelled peptide substrate is attached to said solid support.

A method for identifying at least one specific *Cladosporium* fungus in a sample, comprising the steps of:
a) contacting the sample with a detectably labelled peptide substrate consisting of an amino acid sequence selected from the group consisting of:
   KAAHKSALKSAE (SEQ ID NO: 1),
   ACCDEYLQTKE (SEQ ID NO: 4),
   KLPHKLSWSADNP (SEQ ID NO: 6) and
   KQNMLSEVERADTE (SEQ ID NO: 8);
      wherein said substrate is specific for at least one characteristic protease produced by the specific *Cladosporium* fungus, under conditions that result in modification of the substrate by the characteristic protease; and
b) detecting the modification or the absence of the modification of the substrate, wherein modification of the substrate indicates the presence of the specific fungus, and wherein the absence of modification of the substrate indicates the absence of the specific fungus, thereby identifying a specific fungus in the sample.

Preferred embodiments are defined in dependent claims 2-6 and 8 to 12.

In the following references to methods for detecting or identifying fungi and molds other than *Cladosporium* do not fall under the claimed invention and are present only as reference examples.

It has been found that the molecules secreted or expressed on the surface of fungal cells, for example enzymes or proteins, can serve as markers for the detection of the presence or absence of a fungus in a sample, for example, the surface of a material containing cellulose or other nutrients necessary for fungal growth.

Accordingly, the present disclosure features a method for detecting the presence or absence of a fungus in a sample, comprising the steps of contacting the sample with a substrate detectably labeled for at least one fungal compound produced by the fungus, under conditions that result in modification of the substrate by the fungal compound; and detecting the modification or the absence of the modification of the substrate, wherein modification of the substrate indicates the presence of the fungus in the sample, and wherein the absence of modification of the substrate indicates the absence of the fungus in said sample.

In other embodiments, this disclosure features a biosensor for detecting the presence or absence of a fungus in a sample, said biosensor comprising a solid support and at least one detectably labeled substrate specific for at least one fungal compound produced by said fungus, said at least one detectably labeled substrate attached to said solid support.

In further embodiments, this disclosure features a kit for detecting a fungus, comprising a biosensor for detecting the presence or absence of said fungus in a sample, said biosensor comprising a solid support and at least one detectably labeled substrate specific for at least one fungal compound produced and/or secreted by said fungus, said at least one detectably labeled substrate attached to said solid support, and one or more reagents for detecting the fungal compound produced and/or secreted by the fungus.

In some embodiments, this disclosure features a method for identifying at least one fungus in a sample, comprising the steps of contacting the sample with a substrate detectably labeled for at least one specific fungal compound produced by a fungus, under conditions that result in modification of the substrate by the specific fungal compound; and detecting the modification or the absence of the modification of the substrate, wherein modification of the substrate indicates the presence of the fungus in the sample, and wherein the absence of modification of the substrate indicates the absence of the fungus in said sample.

The disclosed subject-matter has many advantages. It allows for the detection and/or identification of fungi, including toxic molds, in a wide variety of samples. The articles and methods provided by this invention allow for simple, accurate, and reliable identification and/or detection of fungi. Also, this invention provides for cost-effective and time-saving identification and/or detection of fungi.

### BRIEF DESCRIPTION OF THE DRAWINGS

Figure 1 is a graph of the relative fluorescence of several target polypeptide substrates over time in samples containing mold supernatant or water control, substrate, and reaction buffer.
Figure 2 is a graph of the relative fluorescence of a target polypeptide substrate (P1) over time in samples containing mold supernatant or water control, substrate, and reaction buffer.
Figure 3 is a graph of the relative fluorescence of a target polypeptide substrate (P2) over time in samples containing mold supernatant or water control, substrate, and reaction buffer.
Figure 4 is a graph of the relative fluorescence of a target polypeptide substrate (P3) over time in samples containing mold supernatant or water control, substrate, and reaction buffer.
Figure 5 is a graph of the relative fluorescence of the peptide labeled papal over time in samples containing mold supernatant or water control, substrate, and reaction buffer.
Figure 6 is a graph of the relative fluorescence of the peptide labeled pala1 over time in samples containing mold supernatant or water control, substrate, and reaction buffer.
Figure 7 is a graph of the relative fluorescence of the peptide labeled papg1 over time in samples containing mold supernatant or water control, substrate, and reaction buffer.

### DETAILED DESCRIPTION OF THE INVENTION

As part of their normal growth processes, many fungi produce a number of peptides, polypeptides, enzymes, and proteins to interact with their growth environment. These fungal compounds have numerous functions including, but not limited to, the release of nutrients, protection against host defenses, cell maintenance, and other, as yet undetermined, functions. Some of these fungal compounds are produced on the fungus's cell surface and are exposed to, and interact with, the extracellular environment. Many of these fungal compounds are specific to the species of fungus that produce them, and as such, can serve as specific markers for the presence of that fungus. A system that can detect the presence of these fungal compounds which are produced and/or secreted can equally serve to indicate the presence of the producing/secreting fungus. Alternatively, a system that can detect the absence of these fungal compounds that are produced and/or secreted can equally serve to indicate the absence of the producing/secreting fungus.

In some embodiments, the disclosure features a method for detecting the presence or absence of a fungus in a sample. In one embodiment, the method comprises the steps of contacting the sample with a substrate detectably labeled for at least one fungal compound produced by the fungus, under conditions that result in modification of the substrate by the fungal compound; and detecting the modification or the absence of the modification of the substrate, wherein modification of the substrate indicates the presence of the fungus in the sample, and wherein the absence of modification of the substrate indicates the absence of the fungus in said sample.

As used herein, the term "fungal compound" refers to any compound expressed on the surface of the fungal cell or secreted into the fungus's environment that causes a disease in an animal (i.e. a mycotoxin) or causes property damage. "Fungal compound" also refers to any peptide, polypeptide, protein, or enzyme produced by a fungus and expressed on the surface of the fungal cell or secreted into the fungus's environment.

A fungal detection test system, as described herein, can be tailored to detect one specific fungus by identifying one or more fungal compounds specific to the fungus to be detected. Alternatively, a test system can be designed to simultaneously identify more than one fungal species (for example, at least 2, at least 5, or at least 10 different fungal species), such as those that commonly grow on cellulose-containing substances or those that commonly grow on/in mammalian bodies. Identifying those fungal compounds that are common to certain classes of fungi, but are not produced by others, is one way to achieve this goal. Such fungal compounds can be identified, for example, with a computer-based bioinformatics screen of the microbial genomic databases.

The present disclosure pertains to the identification of fungal compounds that are specific to fungi that destroy property and/or are capable of causing adverse health effects. The presence of one or more specific fungi can be detected by designing a synthetic substrate that will specifically interact with one or more fungal compounds. These synthetic substrates can be labeled with a detectable label such that under conditions wherein the specific fungal compound(s) react with the synthetic substrate, the synthetic substrates undergo a modification, for example, a visible color change that is observed.

These methods can be applied to detect the presence or absence of one or more fungi, and preferably detects those fungi that are most capable of causing property damage and/or those that cause disease in mammals. Examples of fungi to which the methods described herein can be applied are *Absidia* (for example, *Absidia coerulea, Absidia corymbifera, Absidia cylindrospora, Absidia glauca,* or *Absidia spinosa), Acremonium* (for example, *Acremonium falciforme, Acremonium kiliense,* or *Acremonium recifei), Alternaria* (for example, *Alternaria alternata, Alternaria chartarum, Alternaria chlamydospora, Alternaria dianthicola, Alternaria geophilia, Alternaria infectoria, Alternaria longipes, Alternaria stemphyloides,* or *Alternaria tenuissima), Apophysomyces* (for example, *Apophysomyces elegans*), *Arthroderma* (for example, *Arthroderma insingulare, Arthroderma lenticularum, Arthroderma quadrifidum, Arthroderma simii, Arthroderma uncinatum* or *Arthroderma vanbreuseghemii), Aspergillus* (for example, *Aspergillus aculeatus, Aspergillus alliaceus, Aspergillus alutaceus, Aspergillus atroviolaceus, Aspergillus avenaceus, Aspergillus caesiellus, Aspergillus candidus, Aspergillus carneus, Aspergillus chevalieri, Aspergillus clavato-nanicus, Aspergillus clavatus, Aspergillus conicus, Aspergillus deflectus, Aspergillus fischerianus, Aspergillus flavipes, Aspergillus flavus, Aspergillus fumigatus, Aspergillus glaucus, Aspergillus granulosus, Aspergillus hollandicus, Aspergillus janus, Aspergillus japonicus, Aspergillus nidulans, Aspergillus niger, Aspergillus niger* var. *awamori, Aspergillus niveus, Aspergillus ochraceus, Aspergillus oryzae, Aspergillus penicilloides, Aspergillus reptans, Aspergillus restrictus, Aspergillus rubrobrunneus, Aspergillus sclerotiorum, Aspergillus sejunctus, Aspergillus spinosus, Aspergillus sydowii, Aspergillus tamarii, Aspergillus terreus, Aspergillus tetrazonus, Aspergillus thermomutatus, Aspergillus unguis, Aspergillus ustus,* or *Aspergillus versicolor*), *Aureobasidium* (for example, *Aureobasidium pullulans*), *Basidiobolus* (for example, *Basidiobolus ranarum*), *Beauveria* (for example, *Beauveria alba* or *Beauveria bassiana*), *Bipolaris* (for example, *Bipolaris australiensis, Bipolaris cynodontisc, Bipolaris hawaiiensis,* or *Bipolaris spicifera*), *Blastomyces* (for example, *Blastomyces dermatitidis*), *Botryosphaeria* (for example, *Botryosphaeria rhodina*), *Candida* (for example, *Candida aaseri, Candida albicans, Candida amapae, Candida anatomiae, Candida ancudensis, Candida antillancae, Candida apicola, Candida apis, Candida atlantica, Candida atmosphaerica, Candida auringiensis, Candida austromarina, Candida azyma, Candida beechii, Candida bertae, Candida berthetii, Candida blankii, Candida boidinii, Candida boleticola, Candida bombi, Candida bombicola, Candida buinensis, Candida butyri, Candida cantarellii, Candida caseinolytica, Candida castellii, Candida castrensis, Candida catenulata, Candida chilensis, Candida chiropterorum, Candida chodatii, Candida ciferrii, Candida coipomoensis, Candida conglobata, Candida cylindracea, Candida dendrica, Candida dendronema, Candida deserticola, Candida diddensiae, Candida diversa, Candida drimydis, Candida dubliniensis, Candida edax, Candida entomophila, Candida ergastensis, Candida ernobii, Candida ethanolica, Candida euphorbiae, Candida euphorbiiphila, Candida fabianii, Candida famata, Candida famata* var. *famata, Candida famata* var. *flareri, Candida fennica, Candida fermenticarens, Candida firmetaria, Candida floricola, Candida fluviatilis, Candida freyschussii, Candida friedrichii, Candida fructus, Candida galacta, Candida geochares, Candida glabrata, Candida glaebosa, Candida glucosophila, Candida gropengiesseri, Candida guilliermondii, Candida guilliermondii* var. *guilliermondii, Candida guilliermondii* var. *membranaefaciens, Candida haemulonii, Candida homilentoma, Candida humilis, Candida incommunis, Candida inconspicua, Candida insectalens, Candida insectamans, Candida insectorum, Candida intermedia, Candida ishiwadae, Candida karawaiewii, Candida kefyr, Candida krissii, Candida kruisii, Candida krusei, Candida lactis-condensi, Candida laureliae, Candida lipolytica, Candida llanquihuensis, Candida lodderae, Candida lusitaniae, Candida lyxosophila, Candida magnoliae, Candida maltosa, Candida maris, Candida maritima, Candida melibiosica, Candida membranifaciens, Candida mesenterica, Candida methanosorbosa, Candida milleri, Candida mogii, Candida montana, Candida multigemmis, Candida musae, Candida naeodendra, Candida natalensis, Candida nemodendra, Candida norvegensis, Candida norvegica, Candida odintsovae, Candida oleophila, Candida oregonensis, Candida ovalis, Candida palmioleophila, Candida paludigena, Candida parapsilosis, Candida pararugosa, Candida pelliculosa, Candida peltata, Candida petrohuensis, Candida pignaliae, Candida pini, Candida populi, Candida pseudointermedia, Candida pseudolambica, Candida psychrophila, Candida pulcherrima, Candida quercitrusa, Candida quercuum, Candida railenensis, Candida reukaufii, Candida rhagii, Candida robusta, Candida rugopelliculosa, Candida rugosa, Candida saitoana, Candida sake, Candida salida, Candida salmanticensis, Candida santamariae, Candida sanyacobensis, Candida savonica, Candida schatavii, Candida sequanensis, Candida shehatae, Candida shehatae* var. *Insectosa, Candida shehatae* var. *lignosa, Candida shehatae* var. *shehatae, Candida silvae, Candida silvanorum, Candida silvatica, Candida silvicultrix, Candida solani, Candida sonorensis, Candida sophiae-reginae, Candida sorbophila, Candida sorbosa, Candida sorboxylosa, Candida spandovensis, Candida stellata, Candida succiphila, Candida suecica, Candida tanzawaensis, Candida tapae, Candida techellsii, Candida tenuis, Candida torresii, Candida tropicalis, Candida tsuchiyae, Candida utilis, Candida vaccinii, Candida valdiviana, Candida valida, Candida vanderwaltii, Candida vartiovaarae, Candida versatilis, Candida vini, Candida viswanathii, Candida wickerhamii, Candida xestobii,* or *Candida zeylanoides*), *Capronia* (for example, *Capronia semiimmersa), Conidiobolus* (for example, *Conidiobolus coronatus, Conidiobolus incongruus,* or *Conidiobolus lamprauges*), *Cladophialophora* (for example, *Cladophialophora arxii, Cladophialophora bantiana, Cladophialophora boppii, Cladophialophora carrionii, Cladophialophora devriesii,* or *Cladophialophora modesta*), *Cladosporium* (for example, *Cladosporium cladosporioides, Cladosporium herbarum, Cladosporium oxysporum,* or *Cladosporium sphaerospermum*), *Clavispora* (for example, *Clavispora lusitaniae*), *Coccidioides* (for example, *Coccidioides immitis*), *Cochliobolus* (for example, *Cochliobolus australiensis, Cochliobolus cynodontis, Cochliobolus hawaiiensis, Cochliobolus lunatus,* or *Cochliobolus spiciferus*), *Cokeromyces* (for example, *Cokeromyces recurvatus*), *Coniothyrium* (for example, *Coniothyrium fuckelii*), *Cryptococcus* (for example, *Cryptococcus laurentii, Cryptococcus neoformans, Cryptococcus neoformans* var. *neoformans, Cryptococcus neoformans* var. *gattii,* or *Cryptococcus neoformans* var. *grubii*), *Cunninghamella* (for example, *Cunninghamella bertholletiae, Cunninghamella echinulata,* or *Cunninghamella elegans*), *Curvularia* (for example, *Curvularia brachyspora, Curvularia clavata, Curvularia geniculata, Curvularia lunata, Curvularia lunata* var. *aeria, Curvularia pallescens, Curvularia senegalensis,* or *Curvularia verruculosa*), *Emericella* (for example, *Emericella nidulans, Emericella nivea, Emericella quadrilineata,* or *Emericella unguis), Epicoccum* (for example, *Epicoccum purpurascens*), *Epidermophyton* (for example, *Epidermophyton floccosum* or *Epidermophyton stockdaleae), Exophiala* (for example, *Exophiala castellanii, Exophiala jeanselmei, Exophiala jeanselmei* var. *heteromorpha, Exophiala jeanselmei* var. *lecanii-corni, Exophiala moniliae, Exophila pisciphila, Exophiala salmonis,* or *Exophiala spinifera), Exserohilum* (for example, *Exserohilum longirostratum, Exserohilum mcginnisii,* or *Exserohilum rostratum*), *Fennellia* (for example, *Fennellia flavipes* or *Fennellia nivea*), *Fonsecaea* (for example, *Fonsecaea compacta* or *Fonsecaea pedrosoi*), *Fusarium* (for example, *Fusarium aquaeductuum, Fusarium aquaeductuum* var. *media, Fusarium chlamydosporum, Fusarium coeruleum, Fusarium dimerum, Fusarium incarnatum, Fusarium moniliforme, Fusarium napiforme, Fusarium oxysporum, Fusarium proliferatum, Fusarium sacchari, Fusarium semitectum, Fusarium solani, Fusarium sporotrichoides, Fusarium sub glutinans, Fusarium tabacinum,* or *Fusarium verticillioides*), *Gibberella* (for example, *Gibberella fujikuroi*), *Helminthosporium* (for example, *Helminthosporium solani*), *Histoplasma* (for example, *Histoplasma capsulatum, Histoplasma capsulatum* var. *capsulatum,* or *Histoplasma capsulatum* var. *duboisii*), *Hortaea* (for example, *Hortaea werneckii*), *Hyphopichia* (for example, *Hyphopichia burtonii*), *Issatchenkia* (for example, *Issatchenkia orientalis*), *Kluyveromyces* (for example, *Kluyveromyces marxianus*), *Lacazia* (for example, *Lacazia loboi*), *Lasiodiplodia* (for example, *Lasiodiplodia theobromae*), *Leptosphaeria* (for example, *Leptosphaeria coniothyrium, Leptosphaeria thompkinsii,* or *Leptosphaeria senegalensis*), *Lewia* (for example, *Lewia infectoria*), *Madurella* (for example, *Madurella grisea* or *Madurella mycetomatis*), *Microsporum* (for example, *Microsporum audouinii, Microsporum canis, Microsporum distortum, Microsporum gallinae, Microsporum gypseum, Microsporum ferrugineum, Microsporum distortum, Microsporum nanum, Microsporum canis, Microsporum gypseum, Microsporum cookei,* or *Microsporum vanbreuseghemii*), *Mortierella* (for example, *Mortierella polycephala,* or *Mortierella wolfli*), *Mucor* (for example, *Mucor amphibiorum, Mucor circinelloides, Mucor hiemalis, Mucor indicus, Mucor racemosus,* or *Mucor ramosissimus*), *Mycosphaerella* (for example, *Mycosphaerella tassiana*), *Nectria* (for example, *Nectria haematococca*), *Neosartorya* (for example, *Neosartorya fischeri* or *Neosartorya pseudofischeri*), *Neotestudina* (for example, *Neotestudina* rosatii), *Ochroconis* (for example, *Ochroconis gallopava*), *Paracoccidioides* (for example, *Paracoccidioides brasiliensis*), *Penicillium* (for example, *Penicillium canescens, Penicillium chrysogenum, Penicillium citrinum, Penicillium commune, Penicillium decumbens, Penicillium expansum, Penicillium funiculosum, Penicillium griseofulvum, Penicillium janczewskii, Penicillium janthinellum, Penicillium marneffei, Penicillium purpurogenum, Penicillium rugulosum, Penicillium spinulosum,* or *Penicillium verruculosum*), *Phialophora* (for example, *Phialophora americana, Phialophora europaea, Phialophora gregata, Phialophora parasitica Phialophora repens, Phialophora reptans, Phialophora richardsiae,* or *Phialophora verrucosa*), *Pichia* (for example, *Pichia anomala, Pichia deserticola, Pichia euphorbiae, Pichia euphorbiiphila, Pichia fabianii, Pichia guilliermondii, Pichia norvegensis,* or *Pichia ohmeri*), *Plectosphaerella* (for example, *Plectosphaerella cucumerina*), *Pseudallesheria* (for example, *Pseudallesheria boydii*), *Pyrenochaeta* (for example, *Pyrenochaeta mackinnonii, Pyrenochaeta romeroi,* or *Pyrenochaeta unguis-hominis*), Rhizopus (for example, *Rhizopus arrhizus, Rhizopus arygosporus, Rhizomucor miehei, Rhizopus microsporus, Rhizopus microsporus* var. *microsporus, Rhizopus microsporus* var. *oligosporus, Rhizopus microsporus* var. *rhizopodiformis, Rhizopus nigricans, Rhizomucor pusillus, Rhizopus schipperae, Rhizopus stolonifer,* or *Rhizomucor variabilis*), *Saccharomyces* (for example, *Saccharomyces cerevisiae*), *Saksenaea* (for example, *Saksenaea vasiformis*), *Scedosporium* (for example, *Scedosporium apiospermum* or *Scedosporium prolificans*), *Setosphaeria* (for example, *Setosphaeria rostrata*), *Sporothrix* (for example, *Sporothrix cyanescens* or *Sporothrix schenckii*), *Stephanoascus* (for example, *Stephanoascus ciferrii*), *Stachybotrys* (for example, *Stachybotrys chartarum* or *Stachybotrys echinata*), *Syncephalastrum* (for example, *Syncephalastrum racemosum*), *Trichophyton* (for example, *Trichophyton ajelloi, Trichophyton concentricum, Trichophyton equinum, Trichophyton equinum* var. *autotrophicum, Trichophyton equinum* var. *equinum, Trichophyton fischeri, Trichophyton flavescens, Trichophyton gloriae, Trichophyton gourvilii, Trichophyton interdigitale, Trichophyton kanei, Trichophyton krajdenii, Trichophyton long fusum, Trichophyton megninii, Trichophyton mentagrophytes, Trichophyton mentagrophytes* var. *erinacei, Trichophyton mentagrophytes* var. *interdigitale, Trichophyton mentagrophytes* var. *mentagrophytes, Trichophyton mentagrophytes* var. *nodulare, Trichophyton mentagrophytes* var. *quinckeanum, Trichophyton persicolor, Trichophyton phaseolforme, Trichophyton prolferans, Trichophyton raubitschekii, Trichophyton rubrum, Trichophyton schoenleinii, Trichophyton simii, Trichophyton soudanense, Trichophyton terrestre, Trichophyton tonsurans, Trichophyton tonsurans* var. *sulphureum, Trichophyton tonsurans* var. *tonsurans* subvar. *perforans, Trichophyton vanbreuseghemii, Trichophyton verrucosum, Trichophyton violaceum,* or *Trichophyton yaoundei*), *Wangliella* (for example, *Wangiella dermatitidis*), and *Yarrowia* (for example, *Yarrowia lipolytica*). These fungi comprise only examples of the various fungal genera and species to which this invention can be applied, and are not meant to be exhaustive. This invention can be applied to any presently known fungus, as well as any fungal species discovered in the future.

The fungal compound(s) to be detected using the methods described herein can be any compound produced by a fungus and expressed on the surface of the fungal cell or secreted into its environment. For example, the compound can be a mycotoxin; a property damaging compound; or a peptide, polypeptide, protein, or enzyme.

In order to decrease the probability that this method will produce a false positive, the fungal compound chosen as the basis for the fungal detection will preferably be fungus-specific, that is, one which is not frequently detected outside the presence of the particular fungus or group of fungi whose presence or absence is to be detected. Similarly, a particular combination of fungal compounds can also serve as the basis for detection, so long as that combination is relatively rare enough to provide the practitioner with sufficient certainty that a positive detection is sufficiently probative of the presence or absence of the fungus or fungi. Most preferably, the fungal compound or combination of fungal compounds will be chosen because it has no known natural source other than the particular fungus, or group of fungi, whose presence or absence is to be detected.

Because a relatively small amount of an enzyme is able to catalyze the turnover of a substantial amount of substrate, the fungal compound to be detected will typically be an enzyme secreted or expressed by a fungus. By designing the specific substrate so that it reacts with an enzyme, the testing method made possible by this invention will be more sensitive than one designed to react with a non-enzymatic fungal compound. However, those skilled in the art will recognize that the specific substrate can also be designed to be modified by those non-enzymatic fungal compounds.

Some examples of suitable enzymes chosen as the basis for this test include lysins (enzymes that function to lyse cells, including hemolysin stachlysin); cell wall enzymes (enzymes involved in the synthesis and turnover of fungal cell wall components, including peptidoglycan); proteases (enzymes that specifically or non-specifically cleave peptides, polypeptides, or proteins); hydrolases (enzymes that break down polymeric molecules into subunits); metabolic enzymes (enzymes designed to perform various housekeeping functions of a fungal cell, such as breaking down nutrients into components that are useful to the cell); or virulence enzymes (enzymes that are required by the fungal cell to cause a disease; also referred to herein as virulence factors).

For example, the enzyme which modifies the substrate can be a lipase. It has been discovered that certain fungi secrete lipases into their environment as part of their survival and/or virulence mechanisms. The lipases serve to break down lipids in the growth environment in order to release nutrients. Lipases may also play a role in disarming mammalian host defenses during infection. Synthetic substrates for these secreted enzymes can be employed to detect the presence of those fungi that secrete them. By synthesizing lipids attached to dye moieties, it is possible to create substrates that will change color as they are hydrolyzed by secreted lipases. The dye molecule can be one of many commercially available molecules that are colorless when attached to fatty acids, and change color when the substrate is cleaved by lipase. An example of such a dye is Rhodamine-110 (available from Molecular Probes, Eugene, OR). This color change reaction forms the basis of a fungal sensor, which can be incorporated into products including, but not limited to, heathcare products, such as wound dressings, bandages, sutures, or artificial organ replacements; home building products, such as wallpaper, paint, tile, shingles, or a home testing kit for detecting fungi that grow on dwelling or commercial structures; or agricultural products, such as testing materials for detecting fungi that grow on agricultural crops of on agricultural structures.

In another example, the fungal compound can be an autolytic enzyme. Autolysins are enzymes that degrade peptidoglycan, a component of a cell envelope. Autolytic enzymes serve to break down peptidoglycan, be it that of the parent organism, as part of cell division and turnover functions, or as a means to breakdown cell walls of competing organisms. When labeled with para-nitrophenol, synthetic peptidoglycan subunits (such as, but not limited to, N-acetyl-β-d-glucosaminide) serve as indicators that can form the basis of a fungal sensor.

In yet another example, the fungal compound can be beta galactosidase expressed on the surface of fungal cells. Beta galactosidase can be expressed as a cytoplasmic enzyme involved in the metabolism of lactose as an energy source. A labeled synthetic molecule that acts as a substrate for beta galactosidase, (including, but not limited to ortho nitrophenyl β-D-galactopyranoside (ONPG)) could thus be used as a means of detecting a fungus in the environment.

If the fungus to be detected is *Stachybotrys chartarum*, the enzyme to be detected can be, for example, proteinase stachyrase A or hemolysin stachlysin. The afore mentioned enzymes are only a few examples that can be fungal-specific compounds and serve as the basis for fungal detection. Those skilled in the art will recognize that other types of enzymes, as well as non-enzymatic fungal compounds, can serve as the basis for detecting the presence or absence of a fungus.

Substrates for use in the present disclosure includes any molecule, either synthetic or naturally-occurring, that can interact with a fungal compound. Examples of substrates include those substrates described herein, as well as substrates for enzymes that are known in the art. Some other examples of potential substrates include Alt derived fluorescent peptides, for example, KAAHKSALKSAE (SEQ ID NO: 1), KHLGGGALGGGAKE (SEQ ID NO: 2), KHLGGGALGGGAKE (SEQ ID NO: 3); ACCDEYLQTKE (SEQ ID NO: 4); ADTVEPTGAKE (SEQ ID NO: 5); KLPHKLSWSADNP (SEQ ID NO: 6); PVPSTPPTPSPSTP (SEQ ID NO: 7); KQNMLSEVERADTE (SEQ ID NO: 8); KHLGGGGGAKE (SEQ ID NO: 9); or NEAIQEDQVQYE (SEQ ID NO: 10); fluorescent peptidoglycans, for example, fluorecent-N-acetylglucosamine-[b-1,4-N acetylmuramic acid, fluorescent-N- acetylmuramyl-L-alanine, or fluorescent - lipoteichoic acid (peptidoglycans over-labeled with fluorescein would be quenched from fluorescing, but following hydrolysis by a wound pathogen would fluoresce); and a lipid vesicle containing dye for the detection of hemolysin (many hemolysins form ordered protein complexes that are pore forming toxins, and can be detected by the release of dye from a lipid vesicle followed by diffusion of the dye onto a hydrophobic solid substrate). Such substrates described herein can be obtained from commercial sources, e.g., Sigma (St. Louis, MO), or can be produced, e.g., isolated or purified, or synthesized using methods known to those of skill in the art.

The fungal compounds of the present disclosure can modify the substrates. For example, if the fungal compound is an enzyme, the proteins or polypeptides that compose the substrate can be cleaved by a secreted/expressed enzyme that specifically reacts with that substance. In addition to cleaving the substrate, the fungal compound can detectably modify the substrate in some other way, such as causing the substrate to undergo a conformation change. Those are but two examples of how a fungal compound can modify the substrate; other means of substrate modification by fungal compounds are known to those skilled in the art.

Such modifications can be detected to determine the presence or absence of a fungus in a sample. One method for detecting a modification of a substrate by a fungal compound is to label the substrate with two different dyes, where one serves to quench fluorescence resonance energy transfer (FRET) to the other when the molecules (for example, dyes or colorimetric substances) are in close proximity, and the modification is measured by fluorescence.

FRET is the process of a distance dependent excited state interaction in which the emission of one fluorescent molecule is coupled to the excitation of another. A typical acceptor and donor pair for resonance energy transfer consists of 4-(4-(dimethylamino)phenyl)azo benzoic acid (DABCYL) and 5-[(2-aminoethylamino] naphthalene sulfonic acid (EDANS). EDANS is excited by illumination with light at a wavelength of around 336 nm, and emits a photon with wavelength around 490 nm. If a DABCYL moiety is located within 20 angstroms of the EDANS, this photon will be efficiently absorbed. DABCYL and EDANS will be attached to opposite ends of a peptide substrate. If the substrate is intact, FRET will be very efficient. If the peptide has been cleaved by an enzyme, the two dyes will no longer be in close proximity and FRET will be inefficient. The cleavage reaction can be followed by observing either a decrease in DABCYL fluorescence or an increase in EDANS fluorescence (loss of quenching).

If the substrate to be modified is a protein, peptide, or polypeptide, the substrate can be produced using standard recombinant protein techniques (see for example, Ausubel et al., "Current Protocols in Molecular Biology," John Wiley & Sons, (1998), the entire teachings of which are incorporated herein by reference). In addition, the fungal compounds of the present invention can also be generated using recombinant techniques. Through an ample supply of fungal compound or its substrate, the exact site of modification can be determined, and a more specific substrate of the fungal compound can be defined, if so desired. This substrate can also be used to assay for the presence of a specific fungus.

The substrates are labeled with a detectable label that is used to monitor interactions between the fungal compound and the substrate and detect any substrate modifications, for example, cleavage of the substrate or label resulting from such interactions. In addition to Rhodamine-110 and FRET described previously, other detectable labels include various dyes that can be incorporated into substrates, for example, those described herein, spin labels, antigen or epitope tags, haptens, enzyme labels, prosthetic groups, fluorescent materials, chemiluminescent materials, bioluminescent materials, and radioactive materials. Examples of suitable enzyme labels include horseradish peroxidase, alkaline phosphatase, β-galactosidase, and acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride and phycoerythrin; an example of a chemiluminescent material includes luminol; examples of bioluminescent materials include luciferase, luciferin, and aequorin, and examples of suitable radioactive material include ¹²⁵I, ¹³¹I, ³⁵S, and ³H. Other examples of detectable labels include Bodipy, Pyrene, Texas Red, IAEDANS, Dansyl Aziridine, IATR and fluorescein. Succimidyl esters, isothiocyanates, and iodoacetamides of these labels are also commercially available.

One example of a preferred detectable label is a chromogenic dye that allows monitoring of the fungal compound/substrate interaction. An example of such a dye is para-nitrophenol. When conjugated to a substrate molecule, this dye will remain colorless until the fungal compound modifies the substrate, at which point dye turns yellow. Measuring absorbance at around 415 nm in a spectrophotometer can monitor the progress of the enzyme-substrate interaction.

Additional examples of detectable labels are colorimetric components that produce a change in color when the fungal compound interacts with the substrate, wherein the change in color is detectable within the visible band of the light spectrum (e.g., from ∼700 nm to ∼400 nm). In one example, the substrate comprises two colorimetric components. One of the colorimetric components can be a first color, for example, blue, and a second colorimetric component can be a second color, for example, yellow. When present on the same substrate, the unmodified substrate can appear green. If that same substrate is modified (e.g., such as enzymatic cleavage of the yellow colorimetric component from the substrate) the substrate can appear blue. Hence the modification of the substrate would be signaled by a change in color from green to blue. In another example, the substrate comprises one colorimetric component and is attached to a colored solid support. Modification of the substrate results in detachment of the colorimetric component and the colored solid support becomes more visible.

The colorimetric components, as well as the other detectable labels, can be utilized to produce a detectable signal in numerous ways not specifically recited herein yet still encompassed by this invention. Yet further embodiments are described in U.S. Provisional Application 60/516,688 filed on November 3, 2003, entitled "Colorimetric Substrates, Colorimetric Sensors, and Methods of Use" by Mitchell C. Sanders, Gerard J. Colpas, Diane E. Ellis-Busby, and Jennifer Havard. (The entire teachings of which are incorporated by reference.)

Examples of suitable colorimetric components include reactive dyes and fiber reactive dyes (referred to herein simply as "reactive dyes;" reactive dyes can be colorimetric or fluorescent), are available commercially (from dye manufacturers such as DyStar Textilfarben GmbH & Co. Deutschland KG, Frankfurt, Germany, and chemical companies, such as Sigma Aldrich, Acros, Molecular Probes, and ICN). The type or specific species of dye(s) selected for a detection method, application, or article of manufacture will depend on the properties of the dye (e.g., a molar extinction coefficient) and the environment in which it is to be used.

Reactive Dyes are colored compounds that contain one or two reactive groups capable of forming covalent bonds between the dye and a protein, peptide, substrate, colorimetric components, a solid support, or a collector. Approximately 80% of all reactive dyes are based on the azo chromophore. Fiber reactive dyes are colored compounds that have a reactive group capable of forming a covalent bond with a fiber. These dyes have been historically used in the textile industry.

Examples of other suitable dyes include those that are approved for use in contact lenses and/or sutures by the U.S. Food and Drug Administration (e.g., Reactive Black 5, Reactive Blue 21, Reactive Orange 78, Reactive Yellow 15, Reactive Blue 19, Reactive Blue 4, Reactive Red 11, Reactive Yellow 86, Reactive Blue 163, Reactive Red 180); mono- and dihalogentriazine dyes (e.g., mono- and di-fluorotriazine dyes; mono- and di-chlorotriazine dyes; mono-(m'-carboxypyridinium) triazines; Reactive Blue 4; Reactive Yellow 86; dyes in the PROCION® line of dyes (e.g., PROCION® Blue HB), dyestuffs, and coloring matters, which are available from BASF; and the CIBACRON™ line of coal tar colors, which are available from Ciba-Geigy); 2,4,5 trihalogenopyriminidines; 2,3 dihaloquinoxalines; N-hydroxysulfosuccinimidyl (sulfo-NHS) ester functionalized dyes; N-hydroxysuccinimidyl (NHS) functionalized dyes; vinyl sulfone dyes (e.g., REMAZOL® line of coal tar dyestuffs, such as REMAZOL® Blue, produced by DyStar Textilfarben GmbH & Co. Deutschland KG; and Reactive Black 5); and sulfonyl chloride dyes (e.g., lissamine rhodamine, and dabsyl chloride); tetrafluorophenyl ester functionalized dyes; isothiocyanate functionalized dyes; and iodoacetyl functionalized dyes. Also encompassed by the present invention are dyes that are structurally equivalent to the dyes listed herein.

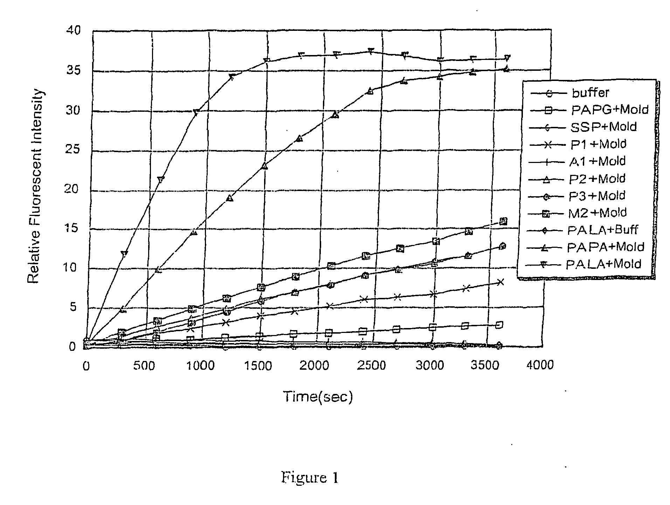

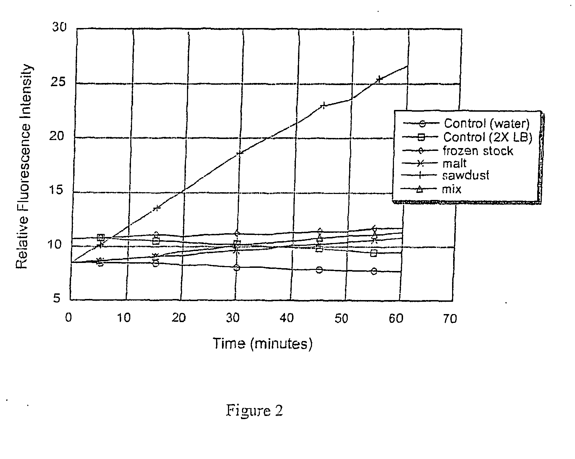

Fiber reactive dyes are based on chlorine or fluorine leaving group chemistries and are known as chloro- or fluoro-triazinyl dyes. Reactive Dyes range from very low reactivity to highly reactive (such as CIBRACRON™ F and PROCION® MX) under a variety of temperature ranges. The reactive group is a triazinyl ring (a six-sided ring with three nitrogens). The reaction is considered a nucleophilic bimolecular substitution mechanism. It is a specific base-catalyzed addition of the nucleophilic functional group of the substrate to the electrophilic center of the reactive group of the dye. Reactive Blue 4 and Reactive Yellow 86 have the following structure: 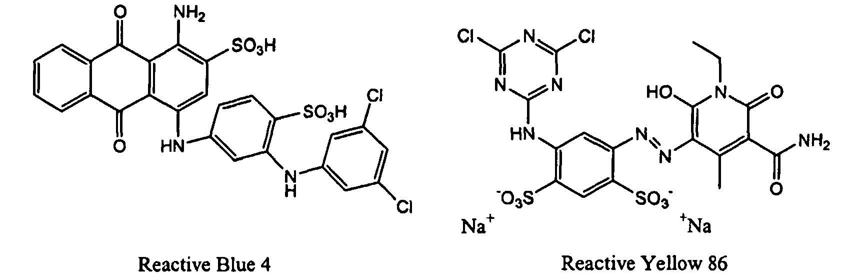 The UV/Visible spectra in water of triazine dye Reactive Blue 4 is illustrated in Figure 1, while the spectra of triazine dye Reactive Yellow 86 is illustrated in Figure 2.

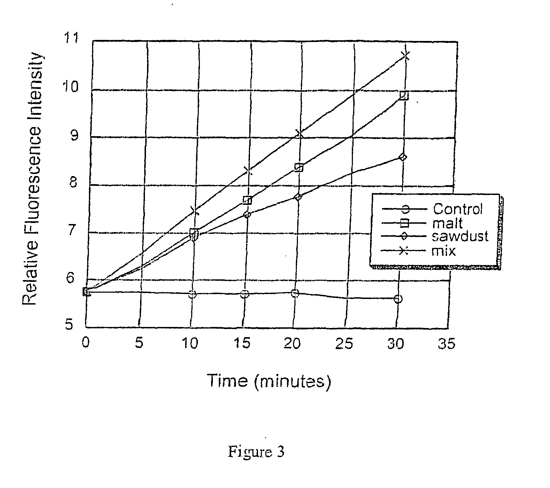

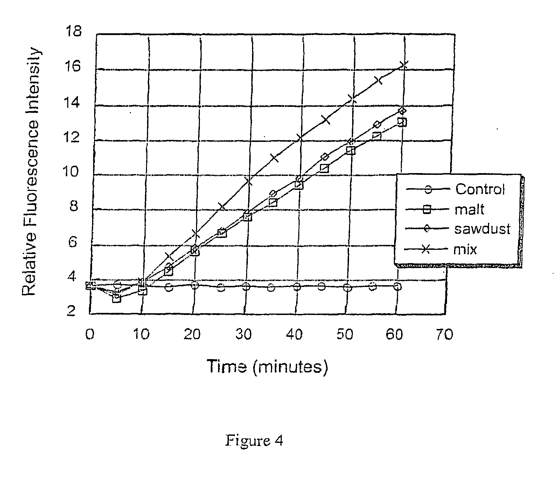

Vinyl sulfone dyes react via a nucleophilic addition mechanism, where there is frequently an elimination step before the addition step, resulting in the formation of a vinylic intermediate. Typically, there is a base-catalyzed elimination of a nucleofugic leaving group followed by a base-catalyzed addition of a nucleophilic functional group of the substrate. REMAZOL® dyes are examples of vinyl sulfone dyes utilizing the reactive group: -SO₂-CH₂-CH₂-OSO₃Na. Reactive Blue 19 and Reactive Black 5 have the following structures: 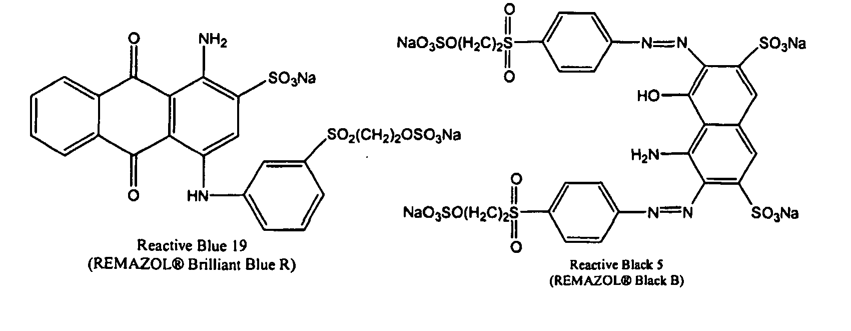 The UV/Visible spectra in water of REMAZOL® Brilliant Blue R is illustrated in Figure 3, while the spectra of REMAZOL® Black B vinyl sulfone is illustrated in Figure 4.

Sulfonyl chlorides are reactive sulfonic acid derivatives. Reaction of sulfonyl chloride compounds with a primary amine-containing molecule proceeds with the loss of chlorine and the formation of a sulfonamide linkage. The structure of the sulfonyl chloride dye, lissamine rhodamine B sulfonyl chloride (i.e., Xanthylium, 9-[4-(chlorosulfonyl)-2-sulfophenyl]-3, 6-bis(diethylamino)-, inner salt, available from Molecular Probes, Inc., Eugene, Oregon, CAS Number/Name: 62796-29-6), is:

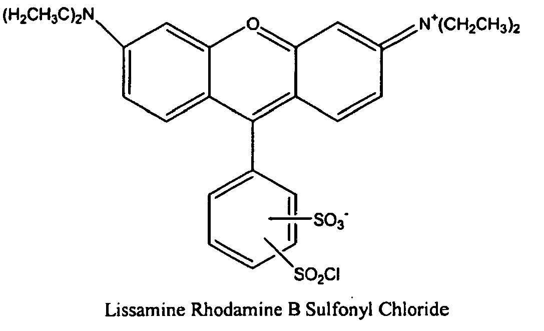

N-hydroxysulfosuccinimidyl (sulfo-NHS) ester functionalized dyes are watersoluble and react with primary amine-containing molecules to form an amide bond with the loss of the sulfo-NHS group. N-hydroxysuccinimidyl (NHS) functionalized dyes are also reactive to amine groups. The structure of the dye functionalized with NHS ester, BODIPY® FL, SSE (i.e., 4,4-difluoro-5,7-dimethyl-4-bora-3a,4a- diaza-*s*-indacene-3-propionic acid, sulfosuccinimidyl ester, sodium salt; available from Molecular Probes, Eugene, Oregon), is:

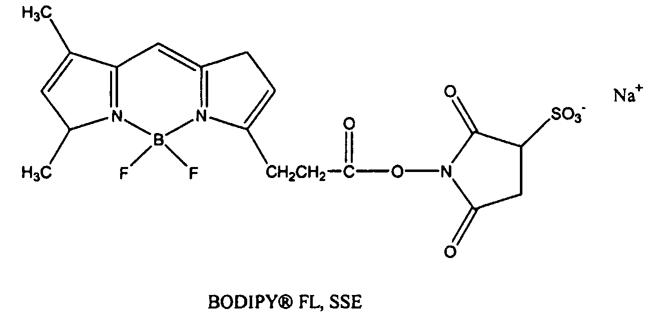

The colorimetric components produce a signal (e.g., a visible change in color) if the substrate is modified (e.g., cleavage of the peptide and/or one or more colorimetric components from the substrate). In this way, the colorimetric components act as a label or tag to indicate the presence or absence of the modification. In some embodiments, the signal is a visible change in color. In other embodiments, the signal is a change in color that is detectable within the visible band of the light spectrum (e.g., from ∼700 nm to ∼400 nm). By attaching a larger number of colorimetric components to the substrate, a more visible color or color change can be produced. In further embodiments, the substrates are labeled with at least two dissimilar colorimetric components. Such embodiments allow for the possibility of producing two or more different changes of hue.

The sample in which the presence or absence of one or more species of fungi is detected can itself be, or can be taken from, virtually any material on which the fungus is suspected of growing. In particular, the sample can be broadly classified into three types: a biological sample, an agricultural sample, or an inanimate sample.

A biological sample can be taken from any living animal, such as birds, reptiles, fish, or mammals, including humans. For example, the biological sample can be a tissue sample; a piece of hair, nail, shell, scale, or feather; or an amount of body fluid, such as blood, urine, sputum, lymph, or wound fluid (for example, pus produced at a wound site).

An agricultural sample can be a plant or plant material on which the fungus is suspected of growing. For example, an agricultural sample can be an amount of an agricultural crop, such as a vegetable, fruit, grain, or hay.

An inanimate sample can be virtually any inanimate object, including a piece of the material on which a fungus is suspected of growing or an object contacted to such a material. For example, the sample can be a piece of building material, such as wallpaper; floor, ceiling, or wall tile; gypsum board; upholstery; fiberboard; building materials made of wood or wood products; insulation materials; drywall; jute; carpet; fabric; siding; paint; a tool.

The sample can also be any article that a fungus can be contained on/in, for example, a catheter, a urine collection bag, a blood collection bag, an article implanted in the mammalian body, a plasma collection bag, a disk, a scope, a filter, a lens, foam, cloth, paper, a suture, swab, test tube, a well of a microplate, or contact lens solutions.

The sample can also be a swab which is contacted with the surface of the material that is to be tested for the presence or absence of the fungus, such as a swab from an area of a room or building, for example, an examination room or operating room of a healthcare facility, a bathroom, a kitchen, a basement, a barn, a grain storage building, or a process or manufacturing facility. The swab can also be contacted with or near parts of the body of an animal, such as the skin, hair, nails, scales, feathers, hide, eyes, ears, nose, mouth, rectum, or any internal organ. Contacting the swab with the body would be especially useful for determining the presence or absence of fungi near areas of a mammalian body that have undergone medical treatment, such as areas where a feeding tube, catheter, heart valve, surgical staples, or sutures have been applied.

In further embodiments, the disclosure includes a wipe. The cleansing wipe includes an absorbent material and one or more sensors. Examples of absorbent material include, but are not limited to, cloth, paper, cotton, sponge, or non-woven fabric material. The wipe is contacted with a surface (e.g., the surface of a wound), and the sensors of the wipe indicate the presence and/or identity of any fungi that may be present. In some embodiments, the sensors are attached to the wipe in the form of a pattern, thereby allowing for a detectable signal that appears in the form of that pattern. In other embodiments, more than one type of sensor is included, so that more than one type of signal is generated to indicate the presence of specific types or species of fungi. Each type of sensor can be arranged in a certain pattern, thereby allowing a specific detectable pattern indicate and distinguish the presence of more than one kind or species of fungi. In still more embodiments, the wipe includes a cleansing or therapeutic material, such as a medicament or an antibiotic.

In some embodiments, this disclosure features a method for identifying at least one fungus in a sample, comprising the steps of contacting the sample with a substrate detectably labeled for at least one specific fungal compound produced by a fungus, under conditions that result in modification of the substrate by the specific fungal compound; and detecting the modification or the absence of the modification of the substrate, wherein modification of the substrate indicates the presence of the fungus in the sample, and wherein the absence of modification of the substrate indicates the absence of the fungus in said sample.

In some embodiments, this disclosure also features a biosensor for detecting one or more (for example, at least 2, at least 5, at least 10, at least 20, at least 30, at least 50, at least 75, or at least 100) fungal compounds described herein and for notifying the user of the presence of the fungal compound. In one embodiment, the biosensor comprises a solid support and at least one detectably labeled substrate specific for at least one fungal compound produced by said fungus, said at least one detectably labeled substrate attached to said solid support. In some embodiments, the substrate is covalently bound to a label and thus has a detection signal that, upon proteolysis of the substrate-label bond, indicates the presence of the fungus.

The biosensor is made by first determining a substrate that is specific for the fungal compound to be detected. The determined specific substrate is labeled with one or more, and preferably, a plurality of detectable labels, for example, chromatogenic or fluorescent leaving groups. Most preferably, the labeling group provides a latent signal that is activated only when the signal is proteolytically detached from the substrate. Chromatogenic leaving groups include, for example, para-nitroanalide groups. Should the substrate come into contact with a fungal compound secreted by a fungus or presented on the surface of a fungal cell, the fungal compound modifies the substrates in a manner that results in detection of such a modification, for example, a change in absorbance, which can be detected visually as a change in color (for example, on the solid support, such as a wound dressing), or using spectrophotometric techniques standard in the art.

The biosensor of the present disclosure also can comprise one or more substrates (for example, at least 2, at least 5, at least 10, at least 20, at least 30, at least 50, at least 75, or at least 100 substrates) for fungal compounds secreted or expressed by one or more species of pathogenic or destructive fungi. The solid support of the biosensor can be virtually any inanimate object, for example, the solid support can be a piece of building material (such as wallpaper; floor, ceiling, or wall tile; gypsum board; upholstery; fiberboard; building materials made of wood or wood products; insulation materials; drywall; jute; carpet; fabric; siding; paint; or a building tool); any article that a fungus may be contained on/in (such as a catheter, a urine collection bag, a blood collection bag, an article implanted in the mammalian body, a plasma collection bag, a disk, a scope, a filter, a lens, foam, cloth, paper, a suture, swab, test tube, a well of a microplate, or contact lens solutions); or a material which is contacted with the surface of an object that is to be tested for the presence or absence of the fungus (such as a swab from an area of a room or building or a swab contacted with or near parts of the body of an animal, such as the skin, hair, nails, scales, feathers, hide, eyes, ears, nose, mouth, rectum, or any internal organ).

Substrates suitably labeled with detectable labels, for example, a chromogenic dye, and attached or incorporated into a sensor apparatus, can act as indicators of the presence or absence of fungi that secrete or express the aforementioned fungal compounds. When more than one substrate is utilized, each can be labeled so as to distinguish it from another (for example, using different detectable labels) and/or each can be localized in a particular region on the solid support.

Substrates with hydrophobic leaving groups can be non-covalently bound to hydrophobic surfaces. Alternatively hydrophilic or hyrdrophobic substrates can be coupled to surfaces by disulfide or primary amine, carboxyl or hydroxyl groups. Methods for coupling substrates to a solid support are known in the art. For example, fluorescent and chromogenic substrates can be coupled to solid substrates using non-essential reactive termini such as free amines, carboxylic acids or SH groups that do not effect the reaction with the fungal compound(s). Free amines can be coupled to carboxyl groups on the substrate using, for example, about a 10 fold molar excess of either N-ethyl-N'-(3-dimethylaminopropyl)carbodiimide hydrochloride (EDC) or N-cyclohexyl-N'-2-(4'-methyl-morpholinium) ethyl carbodiimide-p-toluene sulphonate (CMC) for about 2 hrs at ∼4°C in distilled water adjusted to a pH of ∼4.5 to stimulate the condensation reaction to form a peptide linkage. SH groups can be reduced with DTT or TCEP and then coupled to a free amino group on a surface with N-e-Maleimidocaproic acid (EMCA, Griffith et al., Febs Lett. 134:261-263, 1981).

The biosensors of the present disclosure can be used in any situation where it is desirable to detect the presence or absence of fungi. For example, fungi that grow on, in, or near the bodies of animals; fungi that grow on agricultural products; or fungi that grow on or in inanimate objects, such as a structure. One or more substrates that can be modified by a fungal compound secreted by or presented on the surface of a fungal cell is labeled and covalently bound to a collector substrate, such as cotton fibers on the tip of a swab. When more than one substrate is utilized, each can be labeled so as to distinguish it from another (for example, using different detectable labels) and/or each can be localized in a particular region on the solid support. The swab tip is used to wipe the surface suspected of being contaminated by a fungus. The swab tip is placed in a medium and incubated using conditions that allow modification of the labeled substrate if a fungal compound specific for the bound, labeled substrate(s) is present.

The present disclosure also features a kit for detecting specific fungi. In one embodiment, the kit comprises a biosensor for detecting the presence or absence of a fungus in a sample, said biosensor comprising a solid support and at least one detectably labeled substrate specific for at least one fungal compound produced band/or secreted by said fungus, said at least one detectably labeled substrate attached to said solid support, and one or more reagents for detecting the fungal compound produced and/or secreted by the fungus.

In some embodiments the reagents are in a liquid phase and are specific for fungal detection. In yet further embodiments, the solid support is, for example, a plate having a plurality of wells (e.g., a microtiter plate), to which a detectably labeled substrate is linked, coupled, or attached. In other embodiments, a means for providing one or more buffer solutions is provided. A negative control and/or a positive control can also be provided. Suitable controls can easily be derived by one of skill in the art. For example, a sample suspected of being contaminated by a fungi is prepared using the buffer solution(s). An aliquot of the sample, negative control, and positive control is placed in its own well and allowed to react. Those wells where modification of the substrate, for example, a color change is observed are determined to contain a fungus. Such a kit is particularly useful for determining what species of fungus is growing on the sample being tested or the area where the sample was taken.

The kit that is encompassed by the present disclosure comprises a biosensor and any additional reagents necessary to perform the detection assay. Such a kit can be used to detect the presence or absence of a specific fungus or fungi at the site of fungal contamination, such as inside a structure, in the presence of a mammalian body, or wherever agricultural products are stored, processed, shipped, or consumed.

A method for developing an assay for detecting a fungus that produces at least one fungal compound that is secreted by the cell or present on the surface of the cell and a method for using the assay to detect fungi producing the fungal compound(s) now follows:
Step 1) Define an amino acid sequence that uniquely identifies the fungus of interest. Alternatively a (one or more) amino acid sequence that is unique to a specific group of fungi.
   Select an amino acid sequence, for example, a peptide, polypeptide, protein, or enzyme (marker sequence) that uniquely characterizes or marks the presence of the fungus or group of fungi of interest. The selection can be performed utilizing a bioinformatic approach, for example, as described in detail below. One or more amino acid sequences that are unique to a specific fungus are determined.
Step 2) Obtain sufficient amounts of the fungal compound(s) to determine conditions facilitating optimal modification of a substrate by the fungal compound.
   Isolate the fungal compound from the extracellular medium in which the fungus to be assayed is growing, or from the cell membrane of the fungus, using standard protein purification techniques, described, for example, in Ausubel (supra).
   Alternatively, if the genetic sequence encoding the fungal compound or the location of the genetic sequence encoding the fungal compound are unknown, isolate and clone the genetic sequence encoding the marker amino acid of Step 1, or, first determine the genetic sequence, and then proceed as before.
Step 3) Determine the conditions for growth of the fungus and for the production of a fungal compound presented on the surface of the cell or secreted by the cell.
   Determine the medium required for growth of the specific fungus of interest and for expression of its fungal compound into the medium. Also determine whether a second molecule, for example, an enzyme is required to convert the specific fungal compound from an inactive precursor form to an active form. To determine if the fungal compound has been secreted in an active form, a sample of the fungal culture is provided with chosen potential substrates and cleavage of these substrates is determined. This can be done, for example, by combining the fungus that produce the fungal compound with the substrate in the appropriate media and incubating at room temperature with gentle shaking. At preset times (for example, about 0.1, 0.3, 1.0, 3.0, 5.0, 24 and 48 hours) the samples are centrifuged to spin down the fungi, and a small aliquot is removed for a SDS-PAGE gel sample. After completion of the time course the samples are run on about a 10-15% gradient SDS-PAGE minigel. Then, the proteins are transferred to Immobilon Pseq (Transfer buffer, ∼10% CAPS, ∼10% methanol pH ∼11.0, ∼15 V for ∼30 minutes) using a Bio-Rad semi-dry transblotting apparatus. Following transfer of the proteins, the blot is stained with Coomassie blue R-250 (∼0.25% Coomassie Brilliant Blue R-250, ∼50% methanol, ∼10% acetic acid) and destained (high destain for ∼5 minutes, ∼50% methanol, ∼10% acetic acid; low destain until complete, ∼10% methanol, ∼10% acetic acid) followed by sequencing from the N-terminous. Alternatively, the samples will be run on a mass spectrometer in order to map the sites of proteolytic cleavage using a Voyager Elite Mass spectrometer (Perceptive Biosystems).
Step 4) Identify any specific substrate(s) of the active fungal compound. Examples of potential substrates include proteins, peptides, polypeptides, lipids, and peptidoglycan subunits. Label each substrate with a detectable label, for example, a detectable label described herein, or any other detectable label known in the art.
Step 5) Increase the specificity of the enzyme-substrate interaction (optional) by determining the active or binding site of the fungal compound (for example, using FRET as described above), then determining the genetic sequence useful for producing the active or binding site, and cloning the determined genetic sequence to generate a more specific substrate.
Step 6) Provide a biosensor comprising one or more of the detectably labeled substrates identified above for detection of the fungal compound of the fungus of interest.

The substrate can be attached to a solid support, as described previously. The solid support, if desired, can provide a plurality of derivatized binding sites for coupling to the substrate, for example, succimidyl ester labeled primary amine sites on derivatized plates (Xenobind plates, Xenopore).

Optionally, unoccupied reactive sites on the solid support are blocked by coupling bovine serum albumin, or the active domain of p26 thereto. p26 is described in PCT publication WO 01/83804 A2, November 8, 2001, which is incorporated in its entirety by reference. p26 is an alpha-crystallin type protein that can be used in this case to reduce non specific protein aggregation. The ability of the p26 protein to refold heat denatured citrate synthetase before and after coupling to the surface of the food packaging is used as a control for determining p26 activity. Alpha-crystallin type proteins can be recombinantly produced using standard recombinant DNA technologies (see Ausubel, supra). Briefly, the plasmid containing the beta sheet-charged core domain of p26 is electroporated into electrocompetent BL21 (DE3) cells (Bio-Rad E. coli pulser). The cells are grown up to an OD of ∼0.8, then induced with ∼1mM IPTG for ∼4 hours. The cells are spun down, and sonicated in low buffer (∼10 mM Tris, pH ∼8.0, ∼500 mM NaCl, ∼50mM Imidizole) to lyse (Virsonic, Virtis, Gardiner, NY). The lysate is spun down at ∼13,000 x g for ∼10 minutes, and the supernatant ∼0.45 and ∼0.2 µm filtered. This filtrate is loaded onto a Ni-NTA superose column (Qiagen, Valencia, CA, cat # 30410). High buffer (∼10 mM Tris pH ∼8.0, ∼500 mM NaCl, ∼250 mM Imidizole) is used to elute the protein.

Allow the fungal compound(s) to come into contact with the substrate(s), and monitor the reaction for a modification in the detectably labeled substrate, as described herein. Modification of the substrate indicates that the enzyme produced/secreted by the fungus is present in the reaction, and hence is present on the sample. In addition, the absence of modification of the substrate indicates that the enzyme, and hence the fungus, is not present in the sample.

### EXAMPLES

The present invention will now be illustrated by the following Examples, which are not intended to be limiting in any manner.

### Example 1: Relative Fluorescence of Polypeptide Substrates.

Samples of mold were isolated from several locations in a residential structure. A sample of mold taken from water-damaged dry wall was identified as belonging to the *Cladosporium* genre. Molds isolated from bread were identified as belonging to the genera *Mucor* and *Penicillium.* Molds isolated from a refrigerated piece of cheese and a tomato were identified as belonging to the genre *Cladosporium.* The several *Cladosporium* isolates obtained were not likely from the same species, as they appear to be different in morphology and growing preferences. The various mold samples were grown on a ∼5% malt agar plate with the antibiotics tetracycline, gentamycin, and chloramphenicol.

*A Cladosporium* colony was selected from a plate and grown for several days in a cell culture dish with ∼2 ml of ∼2.5% malt in water on a shaker at room temperature. To allow for oxygen, the cell culture dish was not airtight, and a concentration to one-half volume occurred during growth before being supplemented with water to the original volume of ∼2 ml. An aliquot was taken from the media solution and used to assay for activity. The assay solution consisted of ∼2 µl of the specific peptide solution (∼10 mg/ml in water), ∼8 µl of the *Cladosporium* mold culture media, and ∼90 µl of ∼10 mM tris (pH of ∼7.4) buffer solution. The samples were loaded into individual wells of a microtiter plate and the assay was performed with a fluorimetric plate reader centered at ∼335 nm for excitation and ∼485 nm for emission.

The peptides chosen as substrates were KHLGGGGGAKE (PAPG), NEAIQEDQVQYE (SSP), ACCDEYLQTKE (P1), PVPSTPPTPSPSTP (A1), ADTVEPTGAKE (P2), KLPHKLSWSADNP (P3), KQNMLSEVERADTE (M2), KHLGGGALGGGAKE (PALA), and KAAHKSALKSAE (PAPA). The peptides were labeled with the fluorescent probe EDANS (5-((2-aminoethyl)amino)naphthalene-1- sulfonic acid) on the terminal lysine residue (K) and the quencher dye molecule DABCYL ((4-(4-(dimethylamino)phenyl)azo)benzoic acid) on the terminal glutamate residue (E). These peptides were chosen as substrates because it is known in the art that pathogen-pathogen interactions involve the use of virulence factors to attack and defend against each other. These peptides are known bacterial sequences of virulence factors and can be targets for the mold protease.

FIG. 1 is a graph of the each sample's relative fluorescence over a period of about 60 minutes. As shown in FIG. 1, PAPG and PAPA, mixed with the mold culture media, had the greatest amount of fluorescence, indicating that the fungal compounds in the mold culture media modified those two peptides more readily, relative to the other polypeptide substrates. M2, P2, P3, P1, and PAPG mixed with mold culture media also showed fluorescence activity, indicating they too had been modified by fungal compounds. The blank (buffer only), the control (PALA mixed with buffer only), the SSP substrate mixed with mold culture media, and the A1 substrate mixed with mold culture media showed little or no flourescent activity, indicating a lack of modification.

### Example 2: Varying Media's Effect on Relative Fluorescence of Target Polypeptide Substrate P1

*A Cladosporium* mold culture medium was prepared using the same protocol as previous, except that the media in two of the cell culture dishes contained controls; one being water ("water") and the other 2X Luria Broth ("2X LB"). A third cell culture dish contained frozen stock ("frozen stock"), which is a mold sample stored in a freezer and then thawed. A fourth cell culture dish contained ∼2.5% malt media ("malt"). A fifth cell culture dish contained a sawdust media ("sawdust"), which was used as a substitute for cellulose growth media and was made by adding ∼5% by weight sawdust to water, autoclaving, and then allowing the larger sawdust particles to settle. The upper layer was then used as the growth media. The media in the sixth cell culture dish was a ∼50% mixture of the ∼2.5% malt media and the sawdust media ("mix").

To allow for oxygen, the cell culture dishes were not airtight, and a concentration to about one-half volume occurred during growth before being supplemented with water to the original volume of ∼2 ml. An aliquot was taken from each of the media solutions and used to assay for activity. The assay solution consisted of 2 µl of the P1 peptide solution (∼10 mg/ml in water) as described in Example 1, ∼8 µl of the various mold culture media and controls, and ∼90 µl of ∼10 mM tris (pH ∼7.4) buffer solution. The samples were loaded into individual wells of a microtiter plate and the assay was performed with a fluorimetric plate reader centered at about 335 nm for excitation and about 485 nm for emission.

FIG. 2 is a graph of the each sample's relative fluorescence over a period of about 60 minutes. As shown in FIG. 2, the sample grown in the sawdust media showed the greatest amount of fluorescence. The malt, mix, and frozen stock also showed fluorescent activity, while the two controls showed little or none.

### Example 3: Varying Media's Effect on Relative Fluorescence of Target Polypeptide Substrate P2

*A Cladosporium* colony was grown on a ∼5% malt agar plate with the antibiotics tetracycline, gentamycin, and chloramphenicol. A colony was selected from a plate and grown for several days in cell culture dishes in 2ml of various media, including sawdust and malt, on a shaker at room temperature.

The media contained in the first cell culture dishes was the water control ("water"). A second cell culture dish contained ∼2.5% malt media ("malt"). A third cell culture dish contained a sawdust media ("sawdust"), which was used as a substitute for cellulose growth media and was made by adding ∼5% by weight sawdust to water, autoclaving, and then allowing the larger sawdust particles to settle. The upper layer was then used as the growth media. The media in the fourth cell culture dish was a ∼50% mixture of the ∼2.5% malt media and the sawdust media ("mix").

To allow for oxygen, the cell culture dishes were not airtight, and a concentration to about one-half volume occurred during growth before being supplemented with water to the original volume of ∼2 ml. An aliquot was taken from each of the media solutions and used to assay for activity. The assay solution consisted of ∼2 µl of the P2 peptide solution (∼10 mg/ml in water) as described in Example 1, ∼8 µl of the various mold culture media and controls, and ∼90 µl of ∼10 mM tris (pH ∼7.4) buffer solution. The samples were loaded into individual wells of a microtiter plate and the assay was performed with a fluorimetric plate reader centered at ∼335 nm for excitation and ∼485 nm for emission.

FIG. 3 is a graph of the each sample's relative fluorescence over a period of about 30 minutes. As shown in FIG. 3, all three of the non-control samples exhibited flourescent activity, with the mix media sample fluorescing the most, followed by the malt media sample and the sawdust media sample. The control showed little or no fluorescent activity.

### Example 4: Varying Media's Effect on Relative Fluorescence of Target Polypeptide Substrate P3

*A Cladosporium* colony was grown on a ∼5% malt agar plate with the antibiotics tetracycline, gentamycin, and chloramphenicol. A colony was selected from the plate and grown for several days in cell culture dishes in about 2ml of various media, including sawdust and malt, on a shaker at room temperature.

The media contained in the first cell culture dishes was the water control ("water"). A second cell culture dish contained ∼2.5% malt media ("malt"). A third cell culture dish contained a sawdust media ("sawdust"), which was used as a substitute for cellulose growth media and was made by adding ∼5% by weight sawdust to water, autoclaving, and then allowing the larger sawdust particles to settle. The upper layer was then used as the growth media. The media in the fourth cell culture dish was a ∼50% mixture of the ∼2.5% malt media and the sawdust media ("mix").

To allow for oxygen, the cell culture dishes were not airtight, and a concentration to one-half volume occurred during growth before being supplemented with water to the original volume of ∼2 ml. An aliquot was taken from each of the media solutions and used to assay for activity. The assay solution consisted of ∼2 µl of the P3 peptide solution (∼10 mg/ml in water) as described in Example 1, ∼8 µl of the various mold culture media and controls, and ∼90 µl of ∼10 mM tris (pH ∼7.4) buffer solution. The samples were loaded into individual wells of a microtiter plate and the assay was performed with a fluorimetric plate reader centered at ∼335 nm for excitation and ∼485 nm for emission.

FIG. 4 is a graph of the each sample's relative fluorescence over a period of about 30 minutes. As shown in FIG. 4, all three of the non-control samples exhibited flourescent activity, with the mix media sample fluorescing the most, followed by the sawdust media sample and the malt media sample. The control showed little or no fluorescent activity.

### Example 5: Relative Fluorescence of Target Polypeptide Substrate PAPA, PALA, and PAPG With Various Mold Samples

Various mold samples were prepared with the same protocol as in the previous Examples, except that the mold was grown in a room temperature medium without shaking. The *Cladosporium* species of molds were grown in a ∼2.5% malt extract medium, a ∼2% potato dextrose medium, or a ∼5% sawdust medium. The *Penicillium* molds were grown in malt or potato medium, and the *Mucor* molds were grown in the potato medium.

To allow for oxygen, the cell culture dish was not airtight, and a concentration to one-half volume occurred during growth before being supplemented with water to the original volume of ∼2 ml. An aliquot was taken from the media solution and used to assay for activity. The assay solution consisted of ∼2 µl of the specific peptide solution (∼10 mg/ml in water), ∼8 µl of the mold culture media, and ∼90 µl of ∼10 mM tris (pH ∼7.4) buffer solution. The samples were loaded into individual wells of a microtiter plate and the assay was performed with a fluorimetric plate reader centered at about 335 nm for excitation and about 485 nm for emission.

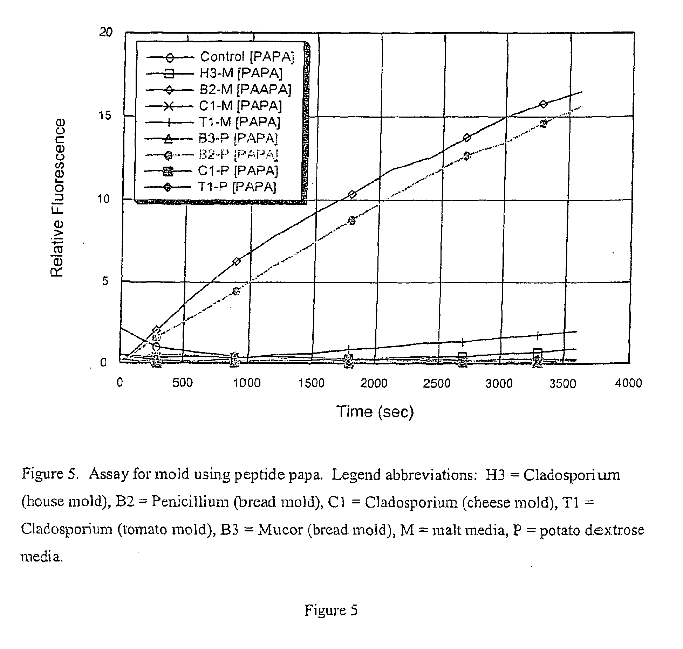

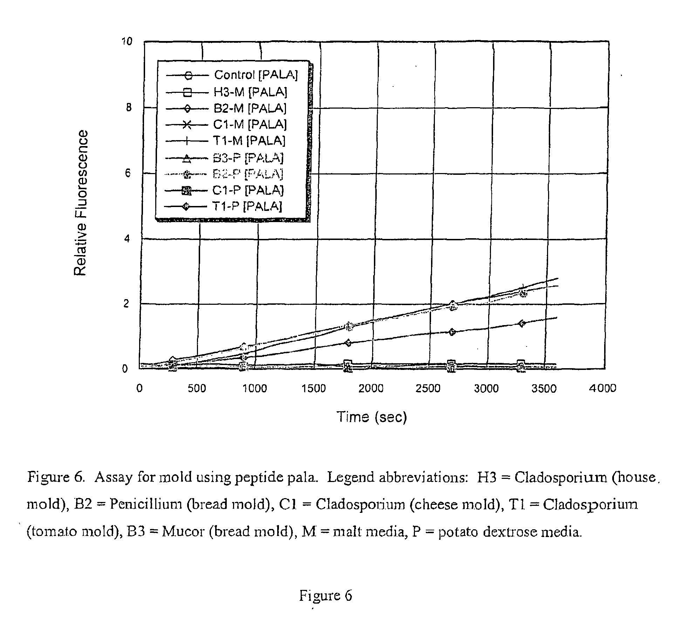

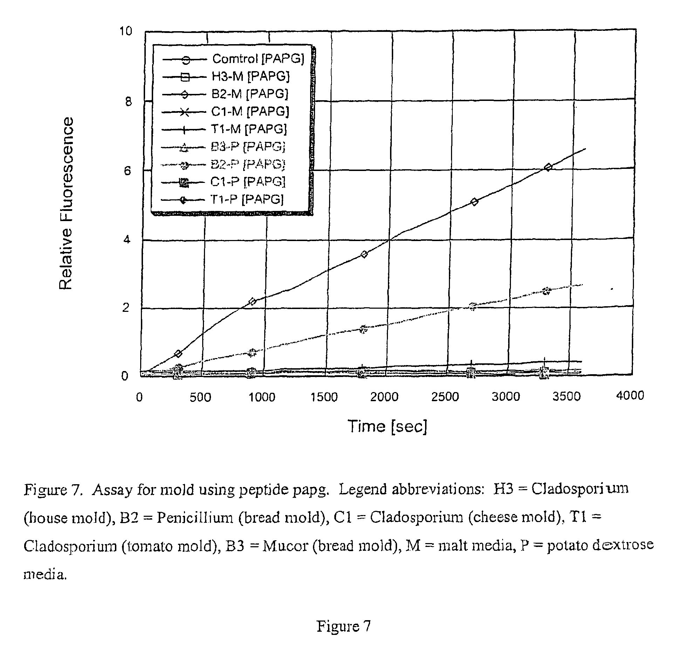

FIG. 5 shows the relative fluorescence with the peptide PAPA. FIG. 6 shows the results obtained with the peptide PALA. FIG. 7 shows the results obtained with the peptide PAPG. The results indicate that PAPA reacts with the *Penicillium* molds grown in malt or potato mediums, as well as with the *Cladosporium* grown in the sawdust or malt mediums. However, the PALA and PAPG peptides showed only a slight reaction with *Penicillium* and none with any of the other molds. This indicates that PALA is a specific assay for *Cladosporium* growing in an environment with similar conditions and nutrients as those found in the sawdust or malt mediums.

## Claims

1. A method for detecting the presence or absence of a *Cladosporium* fungus in a sample, comprising the steps of:
(a) contacting the sample with a detectably labelled peptide substrate consisting ofan amino acid sequence selected from the group consisting of:
KAAHKSALKSAE (SEQ ID NO: 1),
ACCDEYLQTKE (SEQ ID NO: 4),
KLPHKLSWSADNP (SEQ ID NO: 6), and
KQNMLSEVERADTE (SEQ ID NO: 8);
wherein said substrate is specific for at least one fungal protease produced by the fungus to be detected, and wherein the sample is contacted under conditions that result in modification of the substrate by the protease; and
(b) detecting the modification or the absence of the modification of the substrate, wherein modification of the substrate indicates the presence of the fungus in the sample, and wherein the absence of modification of the substrate indicates the absence of the fungus in said sample.

2. The method of Claim 1, wherein said sample is selected from at least one of the group consisting of a biological sample, an agricultural sample, and an inanimate sample.

3. The method of Claim 2, wherein:
a) the biological sample is selected from at least one of the group consisting of a tissue sample, a piece of hair, a piece of nail, a piece of shell, a piece of scale, a piece of feather, and an amount of body fluid;
b) the agricultural sample is selected from at least one of the group consisting of a portion of vegetable, a portion of fruit, a portion of grain, and portion of hay; or
c) the inanimate sample is selected from at least one of the group consisting of a portion of building material, a catheter, a urine collection bag, a blood collection bag, an article implanted in an animal's body, a plasma collection bag, a disk, a scope, a filter, a lens, foam, cloth, paper, a suture, a swab, a test tube, a well of a microplate, and a portion of contact lens solutions.

4. The method according to Claim 1, further comprising the additional step of first growing a portion of the fungus in a fungus culture and sampling said fungus culture to obtain the sample.

5. The method according to Claim 4, wherein the portion of said fungus is grown in a culture media which contains at least one of the group consisting of malt and cellulose.

6. The method according to Claim 1, wherein:
a) the substrate is included in a wipe; or
b) the substrate includes at least one colorimetric component.

7. Use of a biosensor for detecting the presence or absence of a *Cladosporium* fungus in a sample, said biosensor comprising a solid support and a detectably labelled peptide substrate consisting of an amino acid sequence selected from the group consisting of:
KAAHKSALKSAE (SEQ ID NO: 1),
ACCDEYLQTKE (SEQ ID NO: 4),
KLPHKLSWSADNP (SEQ ID NO: 6) and
KQNMLSEVERADTE (SEQ ID NO: 8);
wherein said substrate is specific for a protease produced by the fungus to be detected, and wherein said detectably labelled peptide substrate is attached to said solid support.

8. The use of Claim 7, wherein said sample is selected from at least one of the group consisting of a biological sample, an agricultural sample, and an inanimate sample.

9. The use of claim 8, wherein:
a) the biological sample is selected from at least one of the group consisting of a tissue sample, a piece of hair, a piece of nail, a piece of shell, a piece of scale, a piece of feather, and an amount of body fluid;
b) the agricultural sample is selected from at least one of the group consisting of a portion of vegetable, a portion of fruit, a portion of grain, and portion of hay; or
c) the inanimate sample is selected from at least one of the group consisting of a portion of building material, a catheter, a urine collection bag, a blood collection bag, an article implanted in an animal's body, a plasma collection bag, a disk, a scope, a filter, a lens, foam, cloth, paper, a suture, a swab, a test tube, a well of a microplate, and a portion of contact lens solutions.

10. The use of Claim 7, wherein said solid support comprises a material required to be free of microbial contaminants.

11. The use of Claim 7, wherein said biosensor is contacted directly to an animal body or human body.

12. The use of claim 7, wherein the biosensor is comprised in a kit together with one or more reagents for detecting the protease produced by the *Cladosporium* fungus.

13. A method for identifying at least one specific *Cladosporium* fungus in a sample, comprising the steps of:
a) contacting the sample with a detectably labelled peptide substrate consisting of an amino acid sequence selected from the group consisting of:
KAAHKSALKSAE (SEQ ID NO: 1),
ACCDEYLQTKE (SEQ ID NO: 4),
KLPHKLSWSADNP (SEQ ID NO: 6) and
KQNMLSEVERADTE (SEQ ID NO: 8);
wherein said substrate is specific for at least one characteristic protease produced by the specific *Cladosporium* fungus, under conditions that result in modification of the substrate by the characteristic protease; and
b) detecting the modification or the absence of the modification of the substrate, wherein modification of the substrate indicates the presence of the specific fungus, and wherein the absence of modification of the substrate indicates the absence of the specific fungus, thereby identifying a specific fungus in the sample.

## Patentansprüche

1. Verfahren zum Nachweisen des Vorhandenseins oder des Fehlens eines *Cladosporium-*Fungus in einer Probe, das die folgenden Schritte umfasst:
a) Kontaktieren der Probe mit einem nachweisbar markierten Peptidsubstrat, das aus einer Aminosäurensequenz besteht, die ausgewählt wird aus der Gruppe von:
KAAHKSALKSAE (SEQ ID NO: 1),
ACCDEYLQTKE (SEQ ID NO: 4),
KLPHKLSWSADNP (SEQ NO: 6),
KQNMLSEVERADTE (SEQ ID NO: 8),
wobei das Substrat für zumindest eine durch den nachzuweisenden Fungus produzierte Fungalprotease spezifisch ist und wobei die Probe unter Bedingungen kontaktiert wird, die zu einer Veränderung des Substrats durch die Protease führen, und
b) Nachweisen der Veränderung oder des Fehlens der Veränderung des Substrats, wobei eine Veränderung des Substrats das Vorhandensein des Fungus in der Probe anzeigt und wobei das Fehlen einer Veränderung des Substrats die Abwesenheit des Fungus in der Probe anzeigt.

2. Verfahren nach Anspruch 1, bei dem die Probe von zumindest einer Probe aus der Gruppe ausgewählt wird, die aus einer biologischen Probe, einer landwirtschaftlichen Probe und einer unbelebten Probe besteht.

3. Verfahren nach Anspruch 2, bei dem
a) die biologische Probe von zumindest einem Teil der Gruppe ausgewählt wird, die aus einer Gewebeprobe, einem Stück Haar, einem Stück Nagel, einem Stück äußere Haut, einem Stück Schuppe, einem Stück Feder und einer Menge Körperflüssigkeit besteht,
b) die landwirtschaftliche Probe von zumindest einem Teil der Gruppe ausgewählt wird, die aus einer Portion Gemüse, einer Portion Obst, einer Portion Getreide und einer Portion Heu besteht, oder
c) die unbelebte Probe von zumindest einem Teil der Gruppe ausgewählt wird, die aus einer Portion Baumaterial, einem Katheter, einem Urinsammelbeutel, einem Blutsammelbeutel, einem in einen Tierkörper implantierten Gegenstand, einem Plasmasammelbeutel, einer Scheibe, einem Instrument, einem Filter, einer Linse, Schaum, Tuch, Papier, einer Naht, einem Tupfer, einem Reagenzglas, einer Mulde einer Mikroplatte und einer Portion von Kontaktlinsenlösungen besteht.

4. Verfahren nach Anspruch 1, das ferner den zusätzlichen Schritt umfasst, dass zuerst ein Quantum des Fungus in einer Funguskultur gezüchtet und die Funguskultur zum Erhalt der Probe markiert wird.

5. Verfahren nach Anspruch 4, bei dem das Quantum des Fungus in einem Kulturmedium gezüchtet wird, das zumindest einen Bestandteil der Gruppe enthält, die aus Malz und Zellulose besteht.

6. Verfahren nach Anspruch 1, bei dem
a) das Substrat in einer Wischeinrichtung enthalten ist oder
b) das Substrat zumindest eine kolorimetrische Komponente enthält.

7. Verwendung eines Biosensors zum Nachweis des Vorhandenseins oder des Fehlens eines Cladosporium-Fungus in einer Probe, bei der der Biosensor einen festen Träger und ein nachweisbar markiertes Peptidsubstrat umfasst, das aus einer Aminosäurensequenz besteht, die ausgewählt ist aus der Gruppe von
KAAHKSALKSAE (SEQ ID NO: 1),
ACCDEYLQTKE (SEQ ID NO: 4),
KLPHKLSWSADNP (SEQ ID NO: 6) und
KQNMLSEVERADTE (SEQ ID NO 8),
wobei das Substrat für eine durch den nachzuweisenden Fungus produzierte Protease spezifisch ist und das nachweisbar markierte Peptidsubstrat an dem festen Träger anhaftet.

8. Verwendung nach Anspruch 7, bei der die Probe von zumindest einer Probe aus der Gruppe ausgewählt wird, die aus einer biologischen Probe, einer landwirtschaftlichen Probe und einer unbelebten Probe besteht.

9. Verwendung nach Anspruch 8, bei der
a) die biologische Probe von zumindest einem Teil der Gruppe ausgewählt wird, die aus einer Gewebeprobe, einem Stück Haar, einem Stück Nagel, einem Stück äußere Haut, einem Stück Schuppe, einem Stück Feder und einer Menge Körperflüssigkeit besteht,
b) die landwirtschaftliche Probe von zumindest einem Teil der Gruppe ausgewählt wird, die aus einer Portion Gemüse, einer Portion Obst, einer Portion Getreide und einer Portion Heu besteht, oder
c) die unbelebte Probe von zumindest einem Teil der Gruppe ausgewählt wird, die aus einer Portion Baumaterial, einem Katheter, einem Urinsammelbeutel, einem Blutsammelbeutel, einem in einen Tierkörper implantierten Gegenstand, einem Plasmasammelbeutel, einer Scheibe, einem Instrument, einem Filter, einer Linse, Schaum, Tuch, Papier, einer Naht, einem Tupfer, einem Reagenzglas, einer Mulde einer Mikroplatte und einer Portion von Kontaktlinsenlösungen besteht.

10. Verwendung nach Anspruch 7, bei der der feste Träger ein Material umfasst, das frei von mikrobiellen Verunreinigungen sein soll.

11. Verwendung nach Anspruch 7, bei der der Biosensor direkt mit einem tierischen Körper oder menschlichen Körper kontaktiert wird.

12. Verwendung nach Anspruch 7, bei der der Biosensor in einem Kit zusammen mit einem oder mehreren Reagenzien zum Nachweis der von dem *Cladosporium-*Fungus produzierten Protease enthalten ist.

13. Verfahren zum Identifizieren von zumindest einem spezifischen *Cladosporium-*Fungus in einer Probe, das die folgenden Schritte umfasst:
a) Kontaktieren der Probe mit einem nachweisbar markierten Peptidsubstrat, das aus einer Aminosäurensequenz besteht, die ausgewählt wird aus der Gruppe von
KAAHKSALKSAE (SEQ ID NO: 1),
ACCDEYLQTKE (SEQ ID NO: 4),
KLPHKLSWSADNP (SEQ ID NO: 6) und
KQNMLSEVERADTE (SEQ ID NO 8),
wobei das Substrat für zumindest eine charakteristische Protease spezifisch ist, die von dem spezifischen *Cladosporium*-Fungus unter Bedingungen produziert wird, die zu einer Veränderung des Substrats durch die charakteristische Protease führen, und
b) Nachweisen der Veränderung oder des Fehlens der Veränderung des Substrats, wobei die Veränderung des Substrats das Vorhandensein des spezifischen Fungus anzeigt und wobei das Fehlen der Veränderung des Substrats die Abwesenheit des spezifischen Fungus anzeigt, wodurch ein spezifischer Fungus in der Probe identifiziert wird.

## Revendications

1. Procédé de détection de la présence ou de l'absence d'un champignon *Cladosporium* dans un échantillon, comprenant les étapes consistant à :
(a) mettre l'échantillon en contact avec un substrat peptidique marqué de manière détectable, consistant en une séquence d'acides aminés choisie dans le groupe constitué par :
KAAHKSALKSAE (SEQ ID N° 1),
ACCDEYLQTKE (SEQ ID N° 4),
KLPHKLSWSADNP (SEQ ID N° 6) et
KQNMLSEVERADTE (SEQ ID N° 8) ;
ledit substrat étant spécifique à au moins une protéase fongique produite par le champignon à détecter et le contact avec l'échantillon se faisant dans des conditions qui entraînent une modification du substrat par ladite protéase ; et
(b) détecter la modification ou l'absence de modification du substrat, une modification du substrat indiquant la présence du champignon dans l'échantillon et l'absence de modification du substrat indiquant l'absence du champignon dans ledit échantillon.

2. Procédé selon la revendication 1, dans lequel ledit échantillon est au moins un élément choisi dans le groupe constitué par un échantillon biologique, un échantillon agricole et un échantillon inorganique.

3. Procédé selon la revendication 2, dans lequel :
(a) l'échantillon biologique est au moins un élément choisi dans le groupe constitué par un échantillon tissulaire, un fragment de poil ou cheveu, un fragment d'ongle, un fragment de coquille ou carapace, un fragment d'écaille, un fragment de plume et une quantité de fluide corporel ;
(b) l'échantillon agricole est au moins un élément choisi dans le groupe constitué par une partie de légume, une partie de fruit, une partie de grain et une partie de fourrage ; ou
(c) l'échantillon inorganique est au moins un élément choisi dans le groupe constitué par un morceau de matériau de construction, un cathéter, une poche de collecte d'urine, une poche de collecte de sang, un article implanté dans l'organisme d'un animal, une poche de collecte de plasma, un disque, un microscope, un filtre, une lentille, de la mousse, un textile, du papier, une suture, un écouvillon, un tube à essai, un puits de microplaque et une fraction de solution pour lentilles de contact.

4. Procédé selon la revendication 1, comprenant en plus l'étape consistant à mettre d'abord une partie du champignon en culture dans un milieu de culture fongique et à prélever ledit milieu de culture fongique pour obtenir l'échantillon.

5. Procédé selon la revendication 4, dans lequel ladite partie de champignon est mise en culture dans un milieu de culture contenant au moins un membre du groupe constitué par le malt et la cellulose.

6. Procédé selon la revendication 1, dans lequel :
(a) le substrat est incorporé dans une lingette ; ou
(b) le substrat comprend au moins un composant colorimétrique.

7. Utilisation d'un biocapteur pour détecter la présence ou l'absence d'un champignon *Cladosporium* dans un échantillon, ledit biocapteur comprenant un support solide et un substrat peptidique marqué de manière détectable, consistant en une séquence d'acides aminés choisie dans le groupe constitué par :
KAAHKSALKSAE (SEQ ID N° 1),
ACCDEYLQTKE (SEQ ID N° 4),
KLPHKLSWSADNP (SEQ ID N° 6) et
KQNMLSEVERADTE (SEQ ID N° 8) ;
ledit substrat étant spécifique à une protéase produite par le champignon à détecter et ledit substrat peptidique marqué de manière détectable étant fixé sur ledit support solide.

8. Utilisation selon la revendication 7, dans laquelle ledit échantillon est au moins un élément choisi dans le groupe constitué par un échantillon biologique, un échantillon agricole et un échantillon inorganique.

9. Utilisation selon la revendication 8, dans laquelle :
(a) l'échantillon biologique est au moins un élément choisi dans le groupe constitué par un échantillon tissulaire, un fragment de poil ou cheveu, un fragment d'ongle, un fragment de coquille ou carapace, un fragment d'écaille, un fragment de plume et une quantité de fluide corporel ;
(b) l'échantillon agricole est au moins un élément choisi dans le groupe constitué par une partie de légume, une partie de fruit, une partie de grain et une partie de fourrage ; ou
(c) l'échantillon inorganique est au moins un élément choisi dans le groupe constitué par un morceau de matériau de construction, un cathéter, une poche de collecte d'urine, une poche de collecte de sang, un article implanté dans l'organisme d'un animal, une poche de collecte de plasma, un disque, un microscope, un filtre, une lentille, de la mousse, un textile, du papier, une suture, un écouvillon, un tube à essai, un puits de microplaque et une fraction de solution pour lentilles de contact.

10. Utilisation selon la revendication 7, dans laquelle ledit support solide comprend un matériau qui doit être exempt de contaminants microbiens.

11. Utilisation selon la revendication 7, dans laquelle ledit biocapteur est mis en contact directement avec l'organisme d'un animal ou d'un être humain.

12. Utilisation selon la revendication 7, dans laquelle ledit biocapteur est contenu dans un kit conjointement avec un ou plusieurs réactifs pour détecter la protéase produite par le champignon *Cladosporium.*

13. Procédé d'identification d'au moins un champignon *Cladosporium* spécifique dans un échantillon, comprenant les étapes consistant à :
(a) mettre l'échantillon en contact avec un substrat peptidique marqué de manière détectable, consistant en une séquence d'acides aminés choisie dans le groupe constitué par :
KAAHKSALKSAE (SEQ ID N° 1),
ACCDEYLQTKE (SEQ ID N° 4),
KLPHKLSWSADNP (SEQ ID N° 6) et
KQNMLSEVERADTE (SEQ ID N° 8) ;
ledit substrat étant spécifique à au moins une protéase caractéristique produite par le champignon *Cladosporium* spécifique, dans des conditions entraînant une modification du substrat par ladite protéase caractéristique ; et
(b) détecter la modification ou l'absence de modification du substrat, une modification du substrat indiquant la présence du champignon spécifique et l'absence de modification du substrat indiquant l'absence dudit champignon spécifique, afin d'identifier ainsi un champignon spécifique dans l'échantillon.
